# EUROPEAN PATENT SPECIFICATION

(11) **EP 3 867 644 B1**
(45) Date of publication and mention of the grant of the patent: **21.02.2024**
(21) Application number: 19773182.1
(22) Date of filing: 09.08.2019
(51) Int. Cl.: G01N 33/564, G01N 33/68, G01N 35/00

(54) **SYSTEM AND METHOD FOR SOLID PHASE ANALYSIS OF BIOLOGICAL SAMPLES**
SYSTEM UND VERFAHREN ZUR FESTPHASENANALYSE BIOLOGISCHER PROBEN
SYSTÈME ET PROCÉDÉ POUR L'ANALYSE EN PHASE SOLIDE D'ÉCHANTILLONS BIOLOGIQUES

(30) Priority: 29.08.2018 IT 201800008227; 29.08.2018 IT 201800003292 U
(43) Date of publication of application: 25.08.2021
(73) Proprietor: Cannavale, Giuseppe, 88900 Crotone (KR) (IT)
(72) Inventor: RECINELLI, Alessandro, 00054 FIUMICINO (RM) (IT)
(74) Representative: Karaghiosoff, Giorgio Alessandro
(86) International application number: PCT/IB2019/056797
(87) International publication number: WO 2020/044151

(56) References cited:
- EP-A2- 0 518 827
- M DE FULVIO ET AL: "ENEASYSTEM III: AUTOMAZIONE DEI TEST IgG ED IgG4 SPECIFICHE NELLA DIAGNOSI DI REAZIONI AVVERSE AD ALIMENTI", 37° CONGRESSO NAZIONALE SIBIOC, vol. 29, 14 October 2005 (2005-10-14), pages 215-215, XP055640265,
- V M PLUTINO ET AL: "ENEASYSTEM III: VALUTAZIONE DELLA NUOVA LINEA PER IL TORCH.", 37° CONGRESSO NAZIONALE SIBIOC, vol. 29, 14 October 2005 (2005-10-14), pages 215-215, XP055640268,
- R TOZZOLI ET AL: "Determinazione quantitativa degli autoanticorpi anti-topoisomerasi e anti-proteina B centromerica (CENP B) mediante l'impiego del sistema analitico automatico ENEA System III", RIV MED LAB -JLM, vol. 5, no. 1, 1 January 2004 (2004-01-01) , pages 43-48, XP055640253,
- Bioallergy: "ENEASYSTEM III", , 1 September 2003 (2003-09-01), XP055640138, Retrieved from the Internet: URL:http://www.biosystems.com.ar/archivos/ folletos/107/FOLLETO_ENEASYSTEMIII.pdf [retrieved on 2019-11-07]
- M. PLEBANI ET AL: "Full automation in allergy testing: measurement of specific IgE by the ENEA System", ALLERGY, vol. 50, no. 3, 1 March 1995 (1995-03-01), pages 229-233, XP055640141, United Kingdom ISSN: 0105-4538, DOI: 10.1111/j.1398-9995.1995.tb01139.x

## Description

The present invention is relative to a system and a method for solid phase analysis of biological samples with immunochemical methodology, in order to allow in vitro diagnosis of allergies and autoimmune diseases comprising:
- a plurality of reaction chambers (100) lined and adjacent between them, each reaction chamber being U-shaped and comprising a first vertical branches (101), a second vertical branches (102) and a transverse duct (103) which connects the lower ends of the two branches (101) and (102) together, putting them in fluid communication;
- first feeding means suitable to feed a liquid to be analyzed to the reaction chamber (100) for reacting said liquid with the substances contained in a solid coating, in order to generate immune complexes;
- a washing unit of said reaction chamber (100), able to eliminate the analyte under examination and/or additional reagents fed to the reaction chamber (100);
- second feeding means suitable to feed an additional immuno-enzymatic reagent;
- third feeding means suitable to feed a detection reagent which determines an optically and detectable modification of the immuno-enzymatic reagent in the reaction chamber (100);
   at least one photometric sensor that reads the intensities of optical modifications of the immuno-enzymatic reagent during the reaction with the detector reagent;
- at least one processing unit configured to determine by interpolation with a reference curve, the quantitative numerical values of the allergens and/or antibodies present in the reaction chamber (100) from photometric data;
- a control unit configured to execute a software program in which are coded instructions for controlling said feeding means and said processing units.

As it is known, many types of reaction systems for performing sample analyzes are currently known. In some known systems, a reaction system has a plurality of reaction chambers grouped in the same device and arranged each other with a predetermined order. Systems having reaction chambers are used in combination with fully automatic equipment in which a large number of samples are analyzed, and a possible application of these devices is in 20 vitro analysis of allergies.

Systems for solid phase analysis of biological samples can be used in combination with fully automatic equipment in which a large number of samples are analyzed, said equipment being configured to:
- dispense the samples under examination in the reaction chambers;
- wait the time necessary for the chemical reaction to take place;
- remove by appropriate washing, by mean of an introduction from a first end of a U-chamber duct and suction from the other end of the duct of the unreacted solution;
- feed a tracer reagent;
- wait for the time necessary for the second chemical reaction;
- repeat the washing procedure of the unreacted solution;
- feed an appropriate detection solution;
- wait for the development of the reaction and read the calorimetry or energy variation produced.

In use, the analytes present in biological fluids are generally determined by specific immunological reactions between antibody and antigen that form an immuno-complex.

The reagent that reacts specifically with the analyte is marked so as to allow the detection and measurement of the analyte concentration itself. The marking can be made with radioactive, fluorescent, chemio luminescent substances or by enzymes. In the latter case, the signal relative to the enzyme must be made explicit by adding a suitable substrate which must modify its state so as to become detectable (for example by changing color and energy state) To facilitate the separation between the reacted analyte and the unreacted analyte (so it is not specifically related to the test performed) different solid phases are used. The binding of the specific reagent to the selected solid phase, allows to easily separate the immune complex that is formed between the specific reagent and the analyte. It is possible to use a large number of solid phases, such as: paper discs, plastic sphere, microplate wells, test tubes, cellulose capsules, etc.

A known system is described in patent application US2012/0283122A1, in particular it describes an optimized method, Multiple Microarrays, for dosing multiple and distinct analytes by standardizing an internal calibration curve and a calibration curve with known standards. Two calibration curves are generated during the process of various analytes determination. The system and the method described, uses fluorescent antibodies, each having different wavelength fluorescence, measured by scanner.

Another known method is described in patent application US2011/0306511. The method described concerns dosage and quantification of analytes, distinguishing each analyte, in a single reaction vessel. Two calibration curves are generated during the process of various analytes determination. The method uses different antibodies conjugated with different fluorescence that have different wavelengths for a subsequent identification. Therefore, containing the identification of different analytes in the same reaction chamber.

A system for performing diagnostic assays for allergies and autoimmune diseases is described in WO2014/145581A4. In this patent application, a method and a system are disclosed to improve and overcome some critical issues, in which the analytes present in the biological samples bind to paramagnetic particles for their identification.

Also, methods are known in which it is possible to perform a recalibration of reference curve and curves for families of allergens and/or single allergens on the basis of calibration parameters which can derive from different sources.

Document EP0518827 discloses a system according to the preamble of claim 1. This system is identified by the name ENEAS by Bioallergeny. The said system is further disclosed in the documents:
M DE FULVIO ET AL, "ENEASYSTEM III: AUTOMAZIONE DEI TEST IgG ED IgG4 SPECIFICHE NELLA DIAGNOSI DI REAZIONI AWERSE AD ALIMENTI", 37° CONGRESSO NAZIONALE SIBIOC, (20051014), vol. 29, pages 215 - 215, XP055640265 [X] 1-10;
V M PLUTINO ET AL, "ENEASYSTEM III: VALUTAZIONE DELLA NUOVA LINEA PER IL TORCH.", 37° CONGRESSO NAZIONALE SIBIOC, (20051014), vol. 29, pages 215 - 215, XP055640268 [X] 1-10;
R TOZZOLI ET AL, "Determinazione quantitative degli autoanticorpi anti-topoisomerasi e anti-proteina B centromerica (CENP B) mediante l'impiego del sistema analitico automatico ENEA System III", RIV MED LAB -JLM, (20040101), vol. 5, no. 1, pages 43 - 48, XP055640253 [X] 1-10;
Bioallergy, "ENEASYSTEM III", (20030901), URL: http://www.biosystems.com.ar/archivos/folletos/107/FO LLETO_ENEASYSTEMIII.pdf, (20191107), XP055640138 [X] 1-10;
M. PLEBANI ET AL, "Full automation in allergy testing: measurement of specific IgE by the ENEA System", ALLERGY, United Kingdom, (19950301), vol. 50, no. 3, doi:10.1111/j.1398-9995.1995.tb01139.x, ISSN 0105-4538, pages 229 - 233, XP055640141 [X] 1-10.

Although beneficial in many ways, these systems suffer from the fact that in known reaction chambers the liquid is insert and then aspirated, this procedure, due to the reaction chamber conformation, causes a residual liquid in the reaction chamber, which reduce sensitivity and specificity of the tests.

Purpose of the present invention is to provide a system and method for solid phase analysis of biological samples allowing to optimize a reaction vessel so as to make the production and application phases of the solid phase internal to each reaction chamber, and therefore having characteristics such as to overcome the limits which still influence the systems previously described with reference to the prior art.

According to the present invention, a system for solid phase analysis of biological samples with immunochemical methodology, in order to allow in vitro diagnosis of allergies and autoimmune diseases comprising:
- a plurality of reaction chambers (100) lined and adjacent between them, each reaction chamber being U-shaped and comprising a first vertical branches (101), a second vertical branches (102) and a transverse duct (103) which connects the lower ends of the two branches (101) and (102) together, putting them in fluid communication;
- first feeding means suitable to feed a liquid to be analyzed to the reaction chamber (100) for reacting said liquid with the substances contained in a solid coating, in order to generate immune complexes;
- a washing unit of said reaction chamber (100), able to eliminate the analyte under examination and/or additional reagents fed to the reaction chamber (100);

- second feeding means suitable to feed an additional immuno-enzymatic reagent;
- third feeding means suitable to feed a detection reagent which determines an optically and detectable modification of the immuno-enzymatic reagent in the reaction chamber (100);
   at least one photometric sensor that reads the intensities of optical modifications of the immuno-enzymatic reagent during the reaction with the detector reagent;
- at least one processing unit configured to determine by interpolation with a reference curve, the quantitative numerical values of the allergens and/or antibodies present in the reaction chamber (100) from photometric data;
- a control unit configured to execute a software program in which are coded instructions for controlling said feeding means and said processing unitsCharacterized in that:
   - said at least a reaction chamber (100) comprises an element having at least one of its walls covered with a solid coating in which one or more different substances are contained, and in that said liquid, feed to the reaction chamber (100), reacts with the substances contained in the solid coating, in order to generate immune complexes; and wherein
   - a removable cap for closing the ducts is provided, the cap being made of opaque material having a color univocally related to a specific substances group which can be linked to the solid phase applied or expected in one of the branches of the U-shaped duct, and

   - the said substance groups being the substances contained in the solid phase and, therefore, the analytes that the reaction in each chamber should detect
   - sensors that allow the reading of the colors of the cap is provided for carrying out automatic editing of test results by automatically recognizing the color of the caps associated with the reaction devices.

According to a further feature the said system for solid phase analysis of biological samples is characterized in that characterized in that two rectilinear branches of the U-shaped reaction chamber (100) end with their lower end in a common chamber intended to form a sealing fixing seat of a common closure cap of said chamber.

According to a further feature the above system for solid phase analysis of biological samples according to claim 1 is characterized in that said caps are made of opaque material while the U-shaped reaction chambers (100) are made of transparent material, in order to allow photometric readings.

According to still a further feature the said system for solid phase analysis of biological samples according to claim 1, is characterized in that the first vertical branches (101), and the second vertical branches (102) are rectilinear and parallel, ending in a recess (104) with an elongated cross-section, slot-shaped, said recess (104) being open on the bottom side of the system and constitutes a sealing seat for a lower closure cap that forms, together with a bottom wall of the recess (104), said transverse duct (103) .

According to a further embodiment, the said system for solid phase analysis of biological samples according to claim 1 is characterized in that at two opposite ends of each branches (101, 102) of reaction chambers (100) there are provided means for detachable mutual coupling of an adjacent system which can be provided hooked to one or the other end of a system.

A system for solid phase analysis of biological samples according to claim 1, is further characterized in comprising at a first end a vertical pin (110), and at an opposite end it comprises a tubular seat (120), said pin (110) having a cross-section and a diameter such as to engage the tubular seat, the pin and the tubular seat being provided with a truncated cone- shaped surface and being suitable to be inserted into one another when two systems are coupled together and aligned.

Another embodiment od the system for solid phase analysis of biological samples according to claim 1, is characterized in that said caps (105) is tapered with a truncated cone shape like the recesses (104), and the recesses have at their ends a seat (124) for a further engagement destined to constrain an eccentric axial protrusion (125) of the corresponding caps (105).

Still another embodiment of the system for solid phase analysis of biological samples according to claim 1, is characterized in that the first vertical branches (101) has a diameter smaller than the second vertical branches (102), the transverse duct (103) allowing the passage of liquids from the first branches (101), being a duct with a smaller diameter, to the second branches (102), being a duct with a larger diameter.

According to a further feature, the system for solid phase analysis of biological samples according to claim 1, is characterized in comprising a plurality of reaction chambers (100), made of a transparent rigid material, and a cap (105), made of an opaque elastic material, that can be coupled inserting the protrusion (125) in the seat ( 124 ).

For a better understanding of the present invention, a preferred embodiment is now described, purely by mean of non-limiting example, with reference to the attached drawings, in which:
- figure 1 shows a section view of a system for solid phase analysis of biological samples, according to the invention;
- figure 2 shows another section view of the system for solid phase analysis of biological samples, according to the invention;
- figure 3 shows a top view of the system for solid phase analysis of biological samples, according to the invention;
- figure 4 shows a section view of the system for solid phase analysis of biological samples in figure 4, according to the invention;
- figure 5 shows another section view of the system for solid phase analysis of biological samples of figure 4, according to the invention.

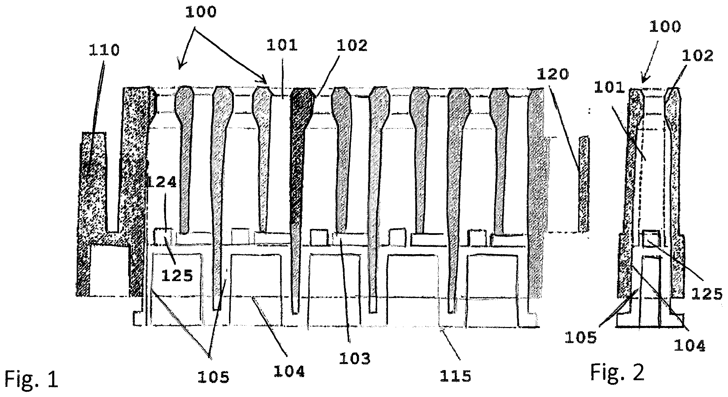

With reference to these figures, and in particular to Figure 1, a system for solid phase analysis of biological samples is shown, according to the invention.

More in detail, the system for solid phase analysis of biological samples comprises:
- At least a reaction chamber 100 consisting in a vertical U-shaped capillary duct;
- First feeding means suitable to feed a liquid to be analyzed, i.e. an analyte under examination, to the reaction chamber 100 for reacting said liquid with the substances contained in the solid coating, in order to generate immune complexes;
- a washing unit of said reaction chamber 100, able to eliminate the analyte under examination and/or additional reagents fed to the reaction chamber at later times in the analysis process;
- second feeding means suitable to feed an additional immuno-enzymatic reagent, for example an enzyme conjugated to the specific antibody for the test analyte;
- third feeding means suitable to feed a detection reagent which determines an optically and detectable modification of the reactants in the reaction chamber 100, in particular of the immuno-enzymatic reagent;
- at least one photometric sensor that reads the intensities of the optical modifications of the immunoenzymatic reagent during the reaction with the detector reagent;
- at least one processing unit configured to determine by interpolation with a reference curve, the quantitative numerical values of the allergens and / or antibodies present in the reaction chamber from the photometric data;
- a control unit configured to execute a software program in which are coded the instructions for controlling said feeding means and said processing units.

According to the invention, the reaction chamber 100 comprises an element having at least one of its walls covered with a solid material in which one or more different substances are contained, as single allergens or autoantigens, or groups or families of allergens or autoantigens.

According to another aspect of the invention, the reaction chamber 100 comprises boundary walls having at least a portion of inner surfaces coated with a solid phase layer containing one or more substances, as single allergens or autoantigens, or groups or families of allergens or autoantigens.

According to an aspect of the invention, at least one of the reaction chambers of the system, preferably each reaction chamber provided in the system, is formed by two parallel and spaced apart ducts, each duct being a branch of the U-shaped reaction chamber. Each duct is open at both ends.

The system further comprises a removable cap 105 for closing the ducts, which can be sealed to one of the two ends of the two parallel tubular ducts. According to an aspect of the invention, the cap 105 is provided with a specific color univocally related to a specific substances group which can be linked to the solid phase applied or expected in one of the branches of the U-shaped duct, such as different families of allergens or different groups of autoantigens.

According to a preferred embodiment, two rectilinear ducts forming the branches of the U-shaped reaction chamber 100 end with their lower end in a common chamber intended to form a sealing fixing seat of a common closure cap of said chamber. The reaction chamber 100 is like a cup while the closure cap 105 has a surface facing the ends of the two ducts that form the branches of the U-shaped reaction chamber 100.

The cap 105 and the chamber take a relative position of end-of-stroke for mutual coupling. In this end-of-stroke position the chamber is sealed together and forms, together with the inner surface of the cap, a transverse conduit connecting the two ducts, forming the two branches of the U-shaped chamber.

According to an aspect of the invention, a reaction container device has a plurality of U-shaped reaction chambers which are arranged aligned with respect to each other, along the pIane defined by U-shaped branches axes. The chambers are spaced from each other in a predetermined extent.

According to an aspect of the invention the liquid flow in the U-shaped reaction chamber is unidirectional, passing through the first and the second branches. This conformation of the reaction chamber, advantageously, allows to optimize the sensitivity and specificity of the process.

Optionally, the lower closing cap of said chambers is constituted by a plurality of side-by-side caps each destined for a U-shaped reaction chamber 100 in which the plurality of caps 105 is integrated in a single block.

Advantageously according to the invention, the colors provided for the caps 105 allow to differentiate the reaction containers of the system on the basis of the substances contained in the solid phase and, therefore, to the analytes that the reaction in each chamber should detect. Therefore, the execution of an analysis aimed at performing immunoassays and identifying and diagnosing allergies or autoimmunities, is facilitated since the service personnel chooses the reaction container having a staining cap corresponding to the family of allergens or groups of autoantigens examined, avoiding errors.

Advantageously, the invention makes it easier to read the test resul ts with reference to the identification of the substances to which a patient's serum has been positive. Since the reading of the test results is done by photometry, the equipment intended to carry out the steps of the procedure are provided with sensors that allow the reading of the colors. It is possible to automate the editing of test resul ts by automatically recognizing the color of the caps associated with the reaction devices.

According to an aspect of the invention, the caps are made of opaque material while the channels forming the U-shaped chambers are made of transparent material, in order to allow photometric readings.

According to an aspect of the invention, the groups of allergens are subdivided and associated with a color according to the following criteria: gramineous - blue; grasses - light green; arboreal plants - dark green; mites - dark orange; house dust - light orange; epithelial - Red; molds and yeasts - light blue; pests, insects, professional poisons - gray; food - Yellow; mixtures of allergens - pink screening test - violet; allergenic components - purple.

Advantageously according to the invention, it is easy to follow the production steps of the reaction container devices with reference to the application in the containers of the solid phases. In fact, having available branches of straight U-shaped channels open at their opposite ends.

Techniques for applying solid phases to the walls of a reaction chamber are known. Generally, walls of the U-shaped chamber are made of a plastic material selected from one or more of the following: plastic polymers such as polystyrene, polyvinyl chloride, polybutadiene, etc., polystyrene-polybutadiene copolymer or glass or a combination of said materials. The walls of at least one branch are coated with the reactant for example by the use of proteins. The coating steps can be preceded by activation steps by, for example, treatment with gamma rays or by plasma treatment or other treatments aimed at improving the adhesion of coating layers.

It is clear that the manipulations necessary for the coating of a duct open on both ends is much easier than that of a practically blind duct, except for a transverse cross-section.

Advantageously according to the invention, the two part construction of the U-shaped chambers also has advantages in terms of manufacture. The two branches can be easily made by drilling further, the realization of ducts open at the two ends avoids the formation of undercuts which would make a manufacturing process by injection molding techniques complex.

According to an aspect of the invention, the system comprises a plurality of reaction chambers 100 in particular lined and adjacent between them, each having a U-shape.

Each U-shaped reaction chamber comprises a first and a second vertical branches 101, 102 and a transverse duct 103 which connects the lower ends of the two vertical branches 101 and 102 together, putting them in fluid communication.

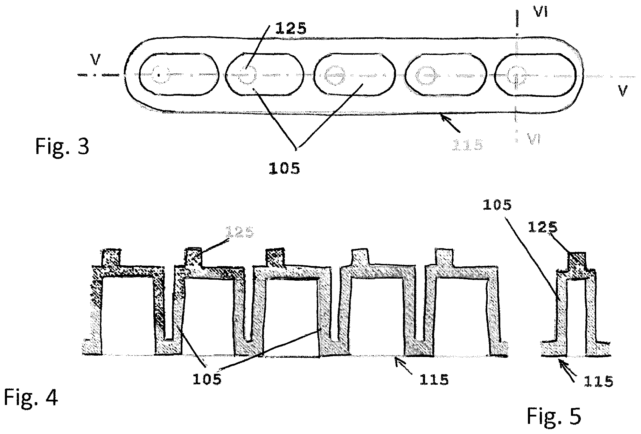

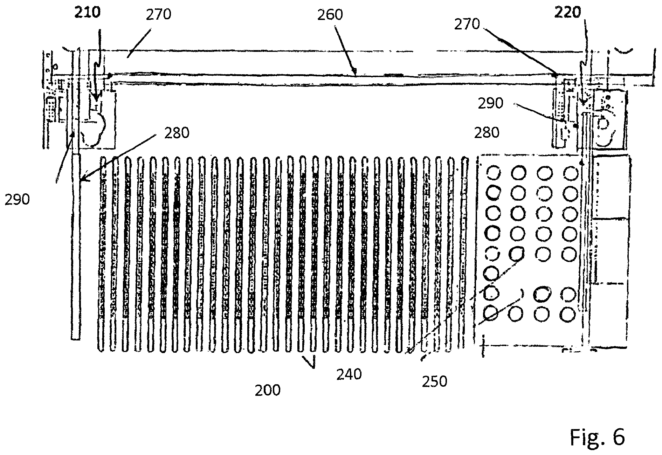

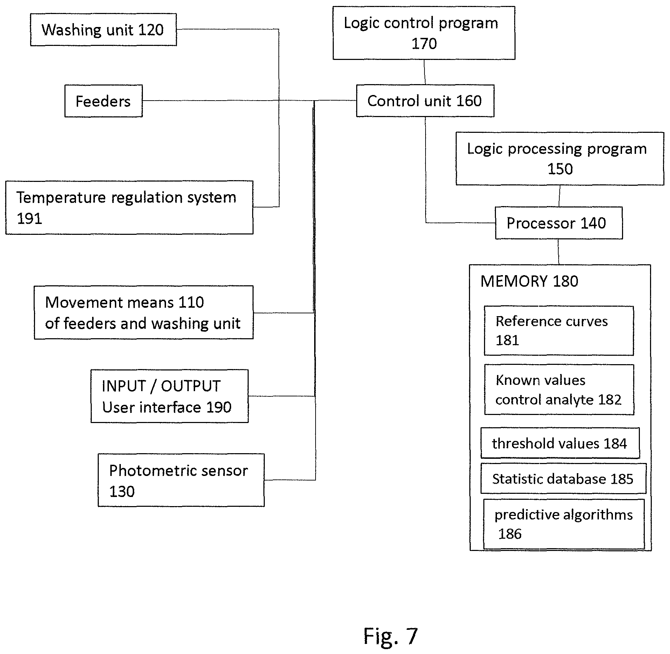

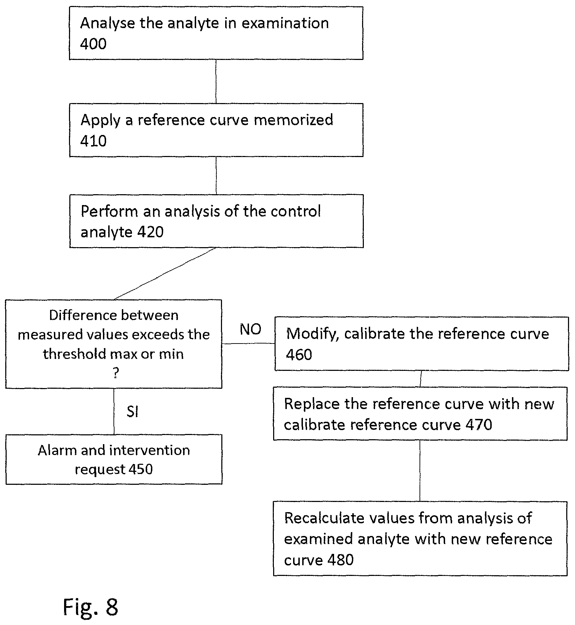

The plurality of reaction chambers 100 are aligned along a vertical plane, sharpened and equally spaced, and with the axes of the vertical branches 101 and 102 of the reaction chambers 100 contained in the same median vertical plane which, with reference to the example illustrated, coincides with the plane V-V of figure 4.

The first and the second branches 101, 102 which form the reaction chambers 100 are preferably rectilinear and parallel, ending in a recess 104 with an elongated crosssection, slot-shaped.

The recess 104 is open on the bottom side of the system and constitutes a sealing seat for a lower closure cap. Said lower closure cap forms, together with the bottom wall of the recess 104, a transverse duct 103 which connects the ends of the two branches 101 and 102 which open into the lower recess 104.

As shown in the figures, each reaction chamber 100 has the same shape as other reaction chambers, and therefore the recesses 104 are identical and open on the bottom side with an identical opening, while the openings are spaced apart.

The caps 105 are provided in a number corresponding to that of the reaction chambers 100 and are part of a single closure body 115 all made in the same material and shaped so as to form individual caps having portions intended to be inserted in the terminal recesses 104 of each reaction chamber 100, while the peripheral walls delimiting such recesses interpenetrate cuneiform cavities intermediate between the individuaI parts of the caps 105.

According to an aspect of the invention, at the two opposite ends of each row of reaction chambers 100 there are provided means for detachable mutual coupling of an adjacent system which can be provided hooked to one or the other end of a system.

According to another aspect of the invention, the system comprises at a first end a vertical pin 110, and at an opposite end 120 it comprises a tubular seat. The pin 110 have a cross-section and a diameter such as to engage the tubular seat.

According to a preferred embodiment, both the pin and the tubular seat are provided with a truncated cone-shaped surface and are intended to be inserted into one another when two systems are coupled together and aligned according to the longitudinal axes along which they are aligned the reaction chambers 100.

According to an aspect of the invention, the caps 105 are tapered with a truncated cone shape like the recesses 104, and the recesses have at their ends a seat 124 for a further engagement destined to constrain an eccentric axial protrusion 125 of the corresponding caps 105.

Advantageously the protrusion 125 and the seat 124 allow a greater coupling force generated between the cap and the recess.

The solid phase of the reactants is at the inner surface of the cavities formed by the ducts forming the reaction chambers 100. Preferably, the reaction chambers are made of Styrolux which is an optically transparent plastic, and is a polystyrene-polybutadiene copolymer. Said material can be easily coated with the solid phase, for example by using proteins, similarly to what happens for wells of a polystyrene microtiter plate.

The reaction chambers 100 can be made alternatively, in materials such as plastic polymers (polystyrene, polyvinyl chloride, polybutadiene, etc.) or glass. For particular purposes, the internal surfaces of the bodies can be activated by treatment with gamma rays, or by plasma treatment or other suitable treatments.

According to a preferred embodiment, the system consists of a parallelepiped device containing five vertical U-shaped reaction chambers 100. The reaction chambers 100 have relatively small diameters and an internal volume of less than 50 µl. Said internal volume is lower than the volume of a common microtiter plate wells.

Preferably, the parallelepiped device containing U-shaped reaction chambers 100 has height equal to 15 mm, comprising the cap in its lower part, and it has height equal to 24mm. the thickness of the parallelepiped device is equal to 3mm in its upper portion and equal to 4mm in its lower portion. The distance between a reaction chamber 100 and a next adjacent reaction chamber 100 is preferably 4.71 mm.

The parallelepiped device has a cylindrical vertical duct at each end, in order to indicate the positioning and inserting direction of the system in the in the analytical platform of an automatic dosing instrument.

According to an aspect of the invention, the first vertical branches 101 has a diameter smaller than the second vertical branches 102. The transverse duct 103 allows the passage of liquids from the first branches 101, being a duct with a smaller diameter, to the second branches 102, being a duct with a larger diameter 102.

According to an aspect of the invention the system comprises a plurality of reaction chambers 100, made of a transparent rigid material, and a cap 105, made of an opaque elastic material, that can be coupled inserting the protrusion 125 in the seat 124. The coupling operation is facilitated by the fact that the cap 105 is made of polyethylene added with a softener, resulting flexible and compressible.

The system for solid phase analysis of biological samples according to the invention is intended for use in an automatic analysis system. The system allows various reagents to be introduced for the assay, including the washing solution between the different steps of the assay, through the first vertical branches 101 of smaller diameter. Instead, the second vertical branches 102, having a larger diameter, is suitable for the suction of fluids after the relative incubation times and also for the photometric reading of the final results of the dosage.

In use, once the system has been placed in the in the test instrument platform, on the coupling pin 110 the tubular element 120 of the subsequent device is inserted. A tubular seat 120 remains free at one end of a row of mutually coupled devices, and a truncated-cone pin 110 remains free at the other end. The tubular seat 120 is supported by a rigid metal driven element which is fixed to the edge of the platform.

Even if the system according to the invention has wide applicability in the field of the detection of analytes in a fluid, it is particularly sui table for immunoassays in solid phase ELISA and analogous immunoassays in solid phase invention. Once the reaction chambers 100 are coated by appropriate procedures with the desired proteins (or other), the proteins (or other) bound to the inner walls remain stable for a few years. The groups of allergens or autoantigens to the walls of the reaction chambers 100 are advantageously indicated by the color of the caps which is uniquely associated with a specific group of these substances.

According to a preferred embodiment, the reaction chambers are in groups of five arranged in a row between each other, each group being connectable to one or more further groups.

According to an aspect of the invention, the system comprises several reaction chambers are provided, preferably grouping in groups of five reaction chambers comprised in an integrated reaction device and at least some of the feeding means and/or washing units can be constituted by a general mean or can be mounted on a general movement system and/or connected to general systems for the distribution of solvents or washing liquids.

Advantageously the system for solid phase analysis of biological samples for analytes present in a liquid solution, especially for in vitro diagnosis of allergies and autoimmune diseases, provide an optimized reaction chamber conformation.

Another advantage of the system and method for solid phase analysis of biological samples is that they allow to optimize a reaction vessel so as to make the production and application phases of the solid phase internal to each reaction chamber easy and effective.

Finally, the system and method for solid phase analysis of biological samples according to the invention is easily achievable and of limited cost with respect to the accessible advantages.

## Claims

1. System for solid phase analysis of biological samples with immunochemical methodology, in order to allow in vitro diagnosis of allergies and autoimmune diseases, comprising:
- a plurality of reaction chambers (100) lined and adjacent between them, each reaction chamber being U-shaped and comprising a first vertical branches (101), a second vertical branches (102) and a transverse duct (103) which connects the lower ends of the two branches (101) and (102) together, putting them in fluid communication;
- first feeding means suitable to feed a liquid to be analyzed to the reaction chamber (100) for reacting said liquid with the substances contained in a solid coating, in order to generate immune complexes;
- a washing unit of said reaction chamber (100), able to eliminate the analyte under examination and / or additional reagents fed to the reaction chamber (100);
- second feeding means suitable to feed an additional immuno-enzymatic reagent;
- third feeding means suitable to feed a detection reagent which determines an optically and detectable modification of the immuno-enzymatic reagent in the reaction chamber (100) ; at least one photometric sensor that reads the intensities of optical modifications of the immuno-enzymatic reagent during the reaction with the detector reagent;
- at least one processing unit configured to determine by interpolation with a reference curve, the quantitative numerical values of the allergens and / or antibodies present in the reaction chamber (100) from photometric data;
- a control unit configured to execute a software program in which are coded instructions for controlling said feeding means and said processing units.
**Characterized in that**:
- said at least a reaction chamber (100) comprises an element having at least one of its walls covered with a solid coating in which one or more different substances are contained, and **in that** said liquid, feed to the reaction chamber (100), reacts with the substances contained in the solid coating, in order to generate immune complexes; and wherein
- a removable cap for closing the ducts is provided, the cap being made of opaque material having a color univocally related to a specific substances group which can be linked to the solid phase applied or expected in one of the branches of the U-shaped duct, and
- the said substance groups being the substances contained in the solid phase and, therefore, the analytes that the reaction in each chamber should detect
- sensors that allow the reading of the colors of the cap is provided for carrying out automatic editing of test results by automatically recognizing the color of the caps associated with the reaction devices.

2. System for solid phase analysis of biological samples according to claim 1, **characterized in that** two rectilinear branches of the U-shaped reaction chamber (100) end with their lower end in a common chamber intended to form a sealing fixing seat of a common closure cap of said chamber.

3. System for solid phase analysis of biological samples according to claim 1, **characterized in that** said caps are made of opaque material while the U-shaped reaction chambers (100) are made of transparent material, in order to allow photometric readings.

4. System for solid phase analysis of biological samples according to claim 1, **characterized in that** the first vertical branches (101), and the second vertical branches (102) are rectilinear and parallel, ending in a recess (104) with an elongated cross-section, slot-shaped, said recess (104) being open on the bottom side of the system and constitutes a sealing seat for a lower closure cap that forms, together with a bottom wall of the recess (104), said transverse duct (103) .

5. System for solid phase analysis of biological samples according to claim 1, **characterized in that** at two opposite ends of each branches (101, 102) of reaction chambers (100) there are provided means for detachable mutual coupling of an adjacent system which can be provided hooked to one or the other end of a system.

6. System for solid phase analysis of biological samples according to claim 1, characterized comprising at a first end a vertical pin (110), and at an opposite end it comprises a tubular seat (120), said pin (110) having a cross-section and a diameter such as to engage the tubular seat, the pin and the tubular seat being provided with a truncated cone- shaped surface and being suitable to be inserted into one another when two systems are coupled together and aligned.

7. System for solid phase analysis of biological samples according to claim 1, **characterized in that** said caps (105) is tapered with a truncated cone shape like the recesses (104), and the recesses have at their ends a seat (124) for a further engagement destined to constrain an eccentric axial protrusion (125) of the corresponding caps (105) .

8. System for solid phase analysis of biological samples according to claim 1, **characterized in that** the first vertical branches (101) has a diameter smaller than the second vertical branches (102), the transverse duct (103) allowing the passage of liquids from the first branches (101), being a duct with a smaller diameter, to the second branches (102), being a duct with a larger diameter.

9. System for solid phase analysis of biological samples according to claim 1, **characterized in** comprising a plurality of reaction chambers (100), made of a transparent rigid material, and a cap (105), made of an opaque elastic material, that can be coupled inserting the protrusion (125) in the seat ( 124 ).

## Patentansprüche

1. System zur Festphasenanalyse von biologischen Proben mit immunochemischer Methodik, um die In-vitro-Diagnose von Allergien und Autoimmunerkrankungen zu ermöglichen, umfassend:
- eine Vielzahl von Reaktionskammern (100), die aneinandergereiht und benachbart zueinander angeordnet sind, wobei jede Reaktionskammer U-förmig ist und einen ersten vertikalen Zweig (101), einen zweiten vertikalen Zweig (102) und einen Querkanal (103) umfasst, der die unteren Enden der beiden Zweige (101) und (102) miteinander verbindet und sie in fluidischer Kommunikation bringt;
- erste Zufuhrmittel, die geeignet sind, eine zu analysierende Flüssigkeit der Reaktionskammer (100) zuzuführen, um diese Flüssigkeit mit den in einer festen Beschichtung enthaltenen Substanzen reagieren zu lassen, um Immunkomplexe zu erzeugen;
- eine Wascheinheit der Reaktionskammer (100), die in der Lage ist, das zu untersuchende Analyt und/oder zusätzliche in der Reaktionskammer (100) zugeführte Reagenzien zu entfernen;
- zweite Zufuhrmittel, die geeignet sind, ein zusätzliches immunoenzymatisches Reagenz zuzuführen;
- dritte Zufuhrmittel, die dazu geeignet sind, ein Nachweisreagenz zuzuführen, das eine optisch und nachweisbare
Modifikation des immunenzymatischen Reagenzes in der Reaktionskammer (100) bestimmt; mindestens einen photometrischen Sensor, der die Intensitäten optischer Modifikationen des immunenzymatischen Reagenzes während der Reaktion mit dem Nachweisreagenz liest;
- mindestens eine Verarbeitungseinheit, die dazu konfiguriert ist, durch Interpolation mit einer Referenzkurve die quantitativen Zahlenwerte der in der Reaktionskammer (100) vorhandenen Allergene und/oder Antikörper aus photometrischen Daten zu bestimmen;
- eine Steuereinheit, die dazu konfiguriert ist, ein Softwareprogramm auszuführen, in dem Anweisungen zur Steuerung der Zufuhrmittel und der Verarbeitungseinheiten codiert sind,
**dadurch gekennzeichnet, dass**
- mindestens eine Reaktionskammer (100) ein Element umfasst, bei dem mindestens eine seiner Wände mit einer festen Beschichtung bedeckt ist, in der eine oder mehrere verschiedene Substanzen enthalten sind, und dass die in der Reaktionskammer (100) zugeführte Flüssigkeit mit den in der festen Beschichtung enthaltenen Substanzen reagiert, um Immunkomplexe zu erzeugen; und wobei
- eine abnehmbare Kappe zum Verschließen der Kanäle vorgesehen ist, wobei die Kappe aus undurchsichtigem Material mit einer Farbe besteht, die eindeutig einer spezifischen Substanzgruppe zugeordnet ist, welche an die in einer der Zweige des U-förmigen Kanals aufgetragenen oder erwarteten festen Phase binden kann, und
- die genannten Substanzgruppen die Substanzen sind, die in der festen Phase enthalten sind und daher die Analyte sind, die die Reaktion in jeder Kammer erkennen soll,
- Sensoren vorgesehen sind, die das Lesen der Kappenfarben ermöglichen, um eine automatische Bearbeitung der Testergebnisse durch automatische Erkennung der den Reaktionsvorrichtungen zugeordneten Farben durchzuführen.

2. System zur Festphasenanalyse von biologischen Proben gemäß Anspruch 1, **gekennzeichnet dadurch, dass** zwei geradlinige Zweige der U-förmigen Reaktionskammer (100) mit ihrem unteren Ende in einer gemeinsamen Kammer enden, die dazu bestimmt ist, einen dichten Befestigungssitz für eine gemeinsame Verschlusskappe dieser Kammer zu bilden.

3. System zur Festphasenanalyse von biologischen Proben gemäß Anspruch 1, **gekennzeichnet dadurch, dass** die Kappen aus undurchsichtigem Material bestehen, während die U-förmigen Reaktionskammern (100) aus transparentem Material bestehen, um photometrische Messungen zu ermöglichen.

4. System zur Festphasenanalyse von biologischen Proben gemäß Anspruch 1, **gekennzeichnet dadurch, dass** die ersten vertikalen Zweige (101) und die zweiten vertikalen Zweige (102) geradlinig und parallel sind und in einer Aussparung (104) mit länglichem schlitzförmigem Querschnitt enden, wobei die Aussparung (104) an der Unterseite des Systems geöffnet ist und einen Dichtsitz für eine untere Verschlusskappe bildet, die zusammen mit einer Bodenwand der Aussparung (104) den Querkanal (103) bildet.

5. System zur Festphasenanalyse von biologischen Proben gemäß Anspruch 1, **gekennzeichnet dadurch, dass** an zwei gegenüberliegenden Enden jedes Zweiges (101, 102) der Reaktionskammern (100) Mittel zur gegenseitigen lösbaren Kopplung eines benachbarten Systems vorgesehen sind, das an einem oder anderen Ende eines Systems angekoppelt werden kann.

6. System zur Festphasenanalyse von biologischen Proben gemäß Anspruch 1, **gekennzeichnet dadurch, dass** an einem ersten Ende ein vertikaler Stift (110) und an einem gegenüberliegenden Ende einen rohrförmigen Sitz (120) vorhanden ist, wobei der Stift (110) einen solchen Querschnitt und Durchmesser hat, dass er in den rohrförmigen Sitz eingreift, wobei der Stift und der rohrförmige Sitz mit einer kegelstumpfförmigen Oberfläche versehen und geeignet sind, ineinander gesteckt zu werden, wenn zwei Systeme miteinander verbunden und ausgerichtet werden.

7. System zur Festphasenanalyse von biologischen Proben gemäß Anspruch 1, **gekennzeichnet dadurch, dass** die Kappen (105) wie die Aussparungen (104) kegelstumpfförmig verjüngt sind und die Aussparungen an ihren Enden einen weiteren Eingriffssitz (124) aufweisen, der zum Einklemmen eines exzentrischen axialen Vorsprungs (125) der entsprechenden Kappen (105) eingerichtet ist.

8. System zur Festphasenanalyse von biologischen Proben nach Anspruch 1, **dadurch gekennzeichnet, dass** die ersten vertikalen Zweige (101) einen kleineren Durchmesser als die zweiten vertikalen Zweige (102) haben, wobei der Querkanal (103) den Durchgang von Flüssigkeiten von den ersten Zweigen (101), die ein Kanal mit einem kleineren Durchmesser sind, zu den zweiten Zweigen (102), die ein Kanal mit einem größeren Durchmesser sind, ermöglicht.

9. System zur Festphasenanalyse von biologischen Proben nach Anspruch 1, **dadurch gekennzeichnet, dass** es eine Vielzahl von Reaktionskammern (100) aus einem transparenten, starren Material und eine Kappe (105) aus einem undurchsichtigen, elastischen Material umfasst, die durch Einsetzen des Vorsprungs (125) in den Sitz (124) gekoppelt werden kann.

## Revendications

1. Système pour l'analyse en phase solide d'échantillons biologiques avec une méthodologie immunochimique, de sorte à permettre diagnostic in vitro d'allergies et de maladies auto-immunes, comprenant:
- une pluralité de chambres de réaction (100) alignées et adjacentes entre elles, chaque chambre de réaction étant en forme de U et comprenant une première branche verticale (101), une seconde branche verticale (102) et un conduit transversal (103) qui relie les extrémités inférieures des deux branches (101) et (102) ensemble, le mettant en communication fluidique;
- premiers moyens d'alimentation appropriés pour introduire un liquide à analyser dans la chambre de réaction (100) de sorte à faire réagir ledit liquide avec les substances contenues dans une couche solide pour générer des complexes immuns;
- une unité de lavage de ladite chambre de réaction (100) capable d'éliminer l'analyte examiné et/ou les réactifs supplémentaires introduits dans la chambre de réaction (100);
- seconds moyens d'alimentation appropriés pour alimenter un réactif immuno-enzymatique supplémentaire;
- troisièmes moyens d'alimentation appropriés pour alimenter un réactif de détection déterminant une modification optique et détectable du réactif immuno-enzymatique dans la chambre de réaction (100); au moins un capteur photométrique qui lit les intensités de modifications optiques du réactif immuno-enzymatique lors de la réaction avec le réactif détecteur;
- au moins une unité de traitement configurée pour déterminer, par interpolation avec une courbe de référence, les valeurs numériques quantitatives des allergènes et/ou anticorps présents dans la chambre de réaction (100) à partir des données photométriques;
- une unité de commande configurée pour exécuter un programme logiciel dans lequel sont codées des instructions pour commander lesdits moyens d'alimentation et lesdites unités de traitement.
**Caractérisé en ce que**:
- ladite au moins une chambre de réaction (100) comprend un élément présentant au moins une de ses parois recouverte d'une couche solide contenant une ou plusieurs substances différentes, et **en ce que** ledit liquide introduit dans la chambre de réaction (100) réagit avec les substances contenues dans la couche solide, de sorte à générer des complexes immuns; et dans lequel
- un capuchon amovible est prévu pour boucher les conduits, le capuchon étant réalisé en un matériau opaque ayant une couleur liée univoquement à un groupe de substances spécifiques pouvant être associées à la phase solide appliquée ou prévue dans l'une des branches du conduit en forme de U, et
- lesdits groupes de substances étant les substances contenues dans la phase solide et, donc, les analytes que la réaction dans chaque chambre devrait détecter,
- capteurs permettant de lire les couleurs du capuchon sont prévus de sorte à réaliser une édition automatique des résultats des tests par reconnaissance automatique de la couleur des capuchons associés aux dispositifs de réaction.

2. Système pour l'analyse en phase solide d'échantillons biologiques selon la revendication 1, **caractérisé en ce que** deux branches rectilignes de la chambre de réaction en forme de U (100) se terminent avec leur extrémité inférieure dans une chambre commune destinée à former un siège de fixation étanche d'un capuchon de fermeture commun de ladite chambre.

3. Système pour l'analyse en phase solide d'échantillons biologiques selon la revendication 1, **caractérisé en ce que** lesdits capuchons sont réalisés en un matériau opaque, tandis que les chambres de réaction en forme de U (100) sont réalisée en un matériau transparent, de sorte à permettre des lectures photométriques.

4. Système pour l'analyse en phase solide d'échantillons biologiques selon la revendication 1, **caractérisé en ce que** les premières branches verticales (101) et les secondes branches verticales (102) sont rectilignes et parallèles, se terminant donc en un renfoncement (104) à section transversale allongée et en forme de fente, ledit renfoncement (104) étant ouvert sur le côté inférieur du système et constituant un siège d'étanchéité pour un capuchon de fermeture inférieur formant, avec une paroi inférieure du renfoncement (104), ledit conduit transversal (103).

5. Système pour l'analyse en phase solide d'échantillons biologiques selon la revendication 1, **caractérisé en ce que**, en correspondance de deux extrémités opposées de chaque branche (101, 102) des chambres de réaction (100), sont prévus des moyens de couplage mutuel détachables d'un système adjacent et pouvant être accrochés à l'une ou à l'autre extrémité d'un système.

6. Système pour l'analyse en phase solide d'échantillons biologiques selon la revendication 1, **caractérisé en ce qu'**il comprend une broche verticale (110) et en correspondance d'une extrémité opposée il comprend un siège tubulaire (120), ladite broche (110) présentant une section transversale et un diamètre tels à engager le siège tubulaire, la broche et le siège tubulaire étant pourvus d'une surface tronconique et étant appropriés pour être insérés l'un dans l'autre lorsque deux systèmes sont couplés ensemble et alignés.

7. Système pour l'analyse en phase solide d'échantillons biologiques selon la revendication 1, **caractérisé en ce que** lesdits capuchons (105) sont effilés et en forme tronconique comme les renfoncements (104), et les renfoncements présentent un siège (124) à leur extrémité pour un engagement supplémentaire destiné à contraindre une saillie axiale excentrique (125) des capuchons correspondants (105).

8. Système pour l'analyse en phase solide d'échantillons biologiques selon la revendication 1, **caractérisé en ce que** les premières branches verticales (101) présentent un diamètre inférieur aux secondes branches verticales (102), le conduit transversal (103) permettant le passage de liquides des premières branches (101), étant un conduit avec un diamètre inférieur, aux secondes branches (102), étant un conduit présentant un diamètre supérieur.

9. Système pour l'analyse en phase solide d'échantillons biologiques selon la revendication 1, **caractérisé en ce qu'**il comprend une pluralité de chambres de réaction (100) réalisées en un matériau transparent rigide et un capuchon (105) réalisé en un matériau élastique opaque, qui peut être couplé en insérant la saillie (125) dans le siège (124).
